# EUROPÄISCHE PATENTSCHRIFT

(11) **EP 2 590 618 B1**
(45) Veröffentlichungstag und Bekanntmachung des Hinweises auf die Patenterteilung: **21.02.2018**
(21) Anmeldenummer: 11729118.7
(22) Anmeldetag: 04.07.2011
(51) Int. Cl.: A61K 8/37, A61Q 5/02, A61K 8/41, A61K 8/42, A61K 8/46

(54) **SCHAUMSTARKES PFLEGESHAMPOO**
HIGHLY FOAMING SHAMPOO
SHAMPOING TRAITANT FORTEMENT MOUSSANT

(30) Priorität: 06.07.2010 DE 102010030983
(43) Veröffentlichungstag der Anmeldung: 15.05.2013
(73) Patentinhaber: Henkel AG & Co. KGaA, 40589 Düsseldorf (DE)
(72) Erfinder: SCHRÖDER, Thomas, 22395 Hamburg (DE); MEYER, Jens, 22880 Wedel (DE); HIPPE, Thomas, 25482 Appen (DE); POPPE, Elisabeth, 22763 Hamburg (DE)
(86) Internationale Anmeldenummer: PCT/EP2011/061203
(87) Internationale Veröffentlichungsnummer: WO 2012/004218

(56) Entgegenhaltungen:
- EP-A1- 1 859 780
- EP-A2- 0 853 941
- US-A- 4 183 952
- US-A- 5 994 280
- US-A1- 2005 164 896

## Beschreibung

Die Erfindung betrifft eine reinigende kosmetische Zusammensetzung, die in einem kosmetischen Träger mindestens ein anionisches Tensid, mindestens zwei amphotere und/oder zwitterionische Tenside sowie mindestens ein Fettsäuremono- und/oder Dialkanolamid enthält. Kosmetische Reinigungsmittel, wie beispielsweise Haarshampoos, basieren auf klassischen anionischen, amphoteren, zwitterionischen, nichtionischen und/oder kationischen Tensiden.

Aufgrund ihres hervorragenden Reinigungs- und Schaumvermögens werden überwiegend anionische Tenside, gegebenenfalls in der Mischung mit geringen Mengen an Co-Tensiden, eingesetzt.

Ein solches handelsübliches Shampoo reinigt die Haare und beseitigt Talg- und/oder Rückstände von Stylingmitteln sowie andere Verschmutzungen von der Haaroberfläche und der Kopfhaut. Während der Reinigung werden aus dem Haar und der Kopfhaut aber auch Lipide und Proteine entfernt, wodurch insbesondere bei häufiger Reinigung eine Schädigung der Haarstruktur und ein Austrocknen der Kopfhaut erfolgen kann.

Um diese Nachteile zu beseitigen wurden daher in den letzten Jahren bevorzugt solche Tenside in Haarreinigungsmitteln eingesetzt, die mild und auf dem Haar sowie der Kopfhaut gut verträglich sind.

Zwar sind an sich milde Tenside bekannt, doch befriedigen diese noch nicht alle Verbraucherwünsche in ausreichendem Maße.

So hat sich beispielsweise gezeigt, dass einige Formulierungen auf der Basis milder Tenside sich nicht immer in der gewünschten Textur bereitstellen lassen. Um die gewünschte Textur zu erhalten, d.h. das gewünschte "cremige" Fließverhalten und eine höhere Viskosität der Mittel einzustellen, sind umfangreiche Formulierungsarbeiten vonnöten, und oft ist die Langzeitstabilität dieser Mittel nicht befriedigend.

Des Weiteren sind milde Tenside oftmals nicht schaumstark und verringern die Schaummenge sowie die Schaumqualität (Cremigkeit und Feinporigkeit des Schaums). Die Einarbeitung von Pflegestoffen in Haarreinigungsmittel kann diesen Effekt noch verstärken.

Schließlich spielt die Haarsensorik bei den Verbrauchern eine herausragende Rolle in Bezug auf die Akzeptanz eines Shampoos.

Neben der Reinigung entscheidet vor allem die Wahrnehmung des Haares nach der Reinigung im nassen und trockenen Zustand über die Zufriedenheit eines Verbrauchers mit einem kosmetischen Haarreinigungsmittel.

Entscheidend bei der Wahrnehmung ist das Gefühl der Pflege, das sich insbesondere durch den Griff, die Kämmbarkeit, den Glanz und die Geschmeidigkeit der Haare beschreiben lässt. Weiterhin sollen die Haare weder schwer erscheinen, noch elektrostatisch aufgeladen sein.

Es besteht daher Weiterhin ein Bedarf nach Wirkstoffen bzw. Wirkstoffkombinationen für kosmetische Reinigungszubereitungen mit guten pflegenden Eigenschaften, einem vorteilhaften Rheologieprofil und guten Schaumeigenschaften.

Der vorliegenden Erfindung lag die Aufgabe zugrunde, ausgewogene pflegende Haarreinigungsmittel mit verbesserter Textur und Schaumqualität bereitzustellen.

Es wurde nun gefunden, dass sich eine spezielle Tensidkombination hervorragend dafür eignet. Die entsprechenden Reinigungsmittel pflegen und reinigen das Haar nicht nur, sondern lassen sich aufgrund ihrer vorteilhaften rheologischen Eigenschaften leicht auf dem Haar verteilen, und bilden in Verbindung mit Wasser einen cremigen, feinporigen Schaum.

Ein erster Gegenstand der Erfindung ist daher eine reinigende kosmetische Zusammensetzung, die in einem kosmetischen Träger - bezogen auf ihr Gewicht -
a) 3 bis 20 Gew.-% mindestens eines anionischen Tensids,
b) 0,05 bis 5 Gew.-% mindestens eines Tensids der Formel (III), 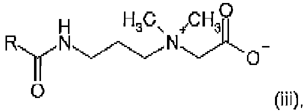 in der R für einen geradkettigen oder verzweigten, gesättigten oder ein- bzw. mehrfach ungesättigten Alkyl- oder Alkenlyrest mit 8 bis 24 Kohlenstoffatomen steht,
c) 0,05 bis 5 Gew.-% mindestens eines Tensids der Formel (v), 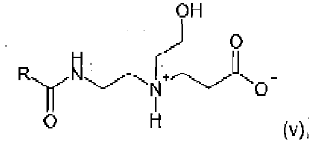 in der R für einen geradkettigen oder verzweigten, gesättigten oder ein- bzw. mehrfach ungesättigten Alkyl- oder Alkenlyrest mit 8 bis 24 Kohlenstoffatomen steht,
d) 0,01 bis 5 Gew.-% mindestens eines C₂-C₄-Mono- und/oder Dialkanolamids mindestens einer C₈-C₂₄-Carbonsäure und
e) 0,0025 bis 1 Gew.-% mindestens eines pflanzlichen, mineralischen, synthetischen oder tierischen Wachses enthält, wobei das Wachs e) Jojobaöl ist.
vgl. Ersatzseite 2a

Die erfindungsgemäßen Zusammensetzungen enthalten die Tenside a) bis c) in einem kosmetischen Träger. Dieser kosmetische Träger im Sinne der Erfindung ist bevorzugt wässrig oder wässrigalkoholisch. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Zusammensetzung, insbesondere Ethanol bzw. Isopropanol, zu verstehen, Ein wässriger Träger enthält im Sinne der Erfindung mindestens 30 Gew.-%, bevorzugt mindestens 40 Gew.-% und insbesondere mindestens 60 Gew,-% Wasser, bezogen auf das Gesamtgewicht der Zusammensetzung.

Die erfindungsgemaßen Zusammensetzungen enthalten als ein erstes Tensid - bezogen auf das Gewicht der Reinigungszusammensetzung - 3 bis 20 Gew.-% mindestens eines anionischen Tensids. Bevorzugt werden die anionischen Tenside in engeren Mengen von 5 bis 15 Gew.-%, mehr bevorzugt von 6 bis 12 Gew.-% und insbesondere von 8 bis 10 Gew.-% - bezogen auf das Gesamtgewicht der Zusammensetzung - eingesetzt.

Erfindungsgemäß bevorzugte anionische Tenside sind
- lineare Alkylsulfonate mit 8 bis 24 C-Atomen,
- lineare Alpha-Olefinsulfonate mit 8 bis 24 C-Atomen,
- Alkylsulfate und Alkylpolyglykolethersulfate der Formel R-O-(CH₂-CH₂O)ₙ-O-SO₃X, in der R bevorzugt für einen geradkettigen oder verzweigten, gesättigten oder ein- bzw. mehrfach ungesättigten Alkyl- oder Alkenlyrest mit 8 bis 24 Kohlenstoffatomen, n für 0 oder 1 bis 12 und X für ein Alkali- oder Erdalkalimetall oder für Triethanolamin steht,
- Sulfonate ungesättigter Fettsäuren mit 8 bis 24 C-Atomen und 1 bis 6 Doppelbindungen. Besonders bevorzugte anionische Tenside sind geradkettige oder verzweigte Alkylethersulfate, die einen Alkylrest mit 8 bis 18 und insbesondere mit 10 bis 16 C-Atomen sowie 1 bis 6 und insbesondere 2 bis 4 Ethylenoxideinheiten enthalten.

Weiterhin besonders bevorzugte anionische Tenside sind geradkettige oder verzweigte Alkylsulfonate, die einen Alkylrest mit 8 bis 18 und insbesondere mit 10 bis 16 C-Atomen enthalten, insbesondere bevorzugt sind die Natrium-, Magnesium und/oder Triethanolaminsalze linearer oder verzweigter Lauryl-, Tridecyl- und/oder Myristylsulfate, die einen Ethoxylierungsgrad von 2 bis 4 aufweisen.

Die erfindungsgemäßen Zusammensetzungen enthalten 0,05 bis 5 Gew-%, vorzugsweise 0,1 bis 4 Gew.-% und insbesondere 0,25 bis 2 Gew.-% Tensid(e) der zuvor genannten Formel (iii) und 0,05 bis 5 Gew.-%, vorzugsweise 0,1 bis 4 Gew.-% und insbesondere 0,25 bis 2 Gew.-% Tensid(e) der zuvor genannten Formel (v).

Die erfindungsgemaßen Zusammensetzungen enthalten als zweites Tensid mindestens eine Verbindung der Formel (iii), in der R für einen geradkettigen oder verzweigten, gesättigten oder ein- bzw. mehrfach ungesättigten Alkyl- oder Alkenlyrest mit 8 bis 24 Kohlenstoffatomen steht.

Diese werden nach der INCI-Nomenklatur als Amidopropylbetaine bezeichnet, wobei die Vertreter, die sich von Kokosfettsäuren ableiten bevorzugt sind und als Cocoamidopropylbetaine bezeichnet werden. Besonders bevorzugt werden erfindungsgemäß Tenside der zuvor genannten Formel (iii) eingesetzt, die ein Gemisch der folgenden Vertreter sind:
H₃C-(CH₂)₇-C(O)-NH-(CH₂)₃N⁺(CH₃)₂CH₂COO⁻
H₃C-(CH₂)₈-C(O)-NH-(CH₂)₃N⁺(CH₃)₂CH₂COO⁻
H₃C-(CH₂)₁₁-C(O)-NH-(CH₂)₃N⁺(CH₃)₂CH₂COO⁻
H₃C-(CH₂)₁₃-C(O)-NH-(CH₂)₃N⁺(CH₃)₂CH₂COO⁻
H₃C-(CH₂)₁₅-C(O)-NH-(CH₂)₃N⁺(CH₃)₂CH₂COO⁻
H₃C-(CH₂)₇-CH=CH-(CH₂)₇-C(O)-NH-(CH₂)₃N⁺(CH₃)₂CH₂COO⁻

Die erfindungsgemäßen Zusammensetzungen enthalten als drittes Tensid mindestens eine Verbindung der zuvor genannten Formel (v), in der R für einen geradkettigen oder verzweigten, gesättigten oder ein- bzw. mehrfach ungesättigten Alkyl- oder Alkenlyrest mit 8 bis 24 Kohlenstoffatomen steht.

Diese Tenside werden nach der 1NCl-Nomenklatur als Amphoacetate bezeichnet, wobei die Vertreter, die sich von Kokosfettsäuren ableiten bevorzugt sind und als Cocoamphoactetate bezeichnet werden.

Tenside dieses Typs können immer auch Betaine der Formel (vi) enthalten, 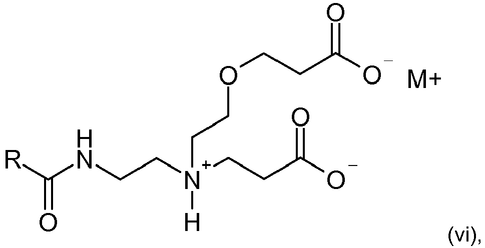 in der R für einen geradkettigen oder verzweigten, gesättigten oder ein- bzw. mehrfach ungesättigten Alkyl- oder Alkenlyrest mit 8 bis 24 Kohlenstoffatomen und M für ein Kation steht. Diese Tenside werden nach der INCI-Nomenklatur als Amphodiacetate bezeichnet, wobei die Vertreter, die sich von Kokosfettsäuren ableiten bevorzugt sind und als Cocoamphodiactetate bezeichnet werden.

Als viertes Tensid enthalten die erfindungsgemäßen Zusammensetzungen - bezogen auf das Gesamtgewicht der Zusammensetzung - 0,01 bis 5 Gew.-% mindestens eines C₂-C₄-Mono- und/oder Dialkanolamids mindestens einer C₈-C₂₄-Carbonsäure.

Bevorzugt wird das C₂-C₄-Mono- und/oder Dialkanolamid in den Zusammensetzungen in einer Menge von 0,025 bis 3 Gew.-%, mehr bevorzugt von 0,05 bis 1,5 Gew.-% und insbesondere von 0,2 bis 1 Gew.-% - bezogen auf das Gesamtgewicht der Zusammensetzung - eingesetzt. Erfindungsgemäß bevorzugte C₂-C₄-Mono- und/oder Dialkanolamide sind die Ethanol- und/oder Isopropanolamide der C₈-C₂₄-Carbonsäuren, wobei die Vertreter, die sich von Kokosfettsäuren, Laurinsäure, Myristinsäure, Palmitinsäure und Stearinsäure ableiten, besonders bevorzugt sind. Erfindungsgemäß besonders bevorzugte C₂-C₄-Mono- und/oder Dialkanolamide werden nach der INCI-Nomenklatur als Cocamide MEA, Cocamide DEA, Cocamide MIPA, Lauramide MEA, Lauramide DEA, Lauramide MIPA, Palmitoylamid MEA, Palmitoylamid DEA, Stearamid MEA und Stearamid DEA bezeichnet.

In einer ersten besonders bevorzugten Ausführungsform der Erfindung enthalten die reinigenden Zusammensetzungen als Tensidkombination a) bis c) mindestens ein geradkettiges oder verzweigtes Lauryl- und/oder Tridecylethersulfat mit einem Ethoxylierungsgrad von 2 bis 4, Cocamidopropylbetain, Cocoamphoacetate oder -diacetate und Cocamide MEA oder Cocamide DEA.

Die gewünschte Textur der erfindungsgemäßen Mittel lässt sich durch die zuvor genannte Tensidkombination erreichen. Die Kombination der Tenside a) bis c) in den erfindungsgemäßen Reinigungszusammensetzungen führt zu den von den Verbrauchern gewünschten cremigfließfähigen, reinigenden und pflegenden Systemen, die schaumstark sind.

Gemäß einer weiteren bevorzugten Ausführungsform der Erfindung können die reinigenden Zusammensetzungen mindestens ein kationisches Polymer enthalten.

Das (die) kationische(n) Polymer(e) wird (werden) in den erfindungsgemäßen Reinigungszusammensetzungen - bezogen auf deren Gesamtgewicht - bevorzugt in Mengen von 0,01 bis 2 Gew.-%, vorzugsweise von 0,02 bis 1 Gew.-% und insbesondere in Mengen von 0,025 bis 0,8 Gew.-% eingesetzt.

Unter erfindungsgemäß geeigneten kationischen Polymeren sind bevorzugt Polymere zu verstehen, welche in der Haupt- und/oder Seitenkette Gruppen aufweisen, welche "temporär" oder "permanent" kationisch sein können. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen enthalten quartäre Ammoniumgruppen.

Erfindungsgemäß bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate,
- hydrophob modifizierte Cellulosederivate, beispielsweise die unter dem Handelsnamen SoftCat® vertriebenen kationischen Polymere,
- kationische Alkylpolyglycoside,
- kationiserter Honig, beispielsweise das Handelsprodukt Honeyquat® 50,
- kationische Guar-Derivate, wie insbesondere die unter den Handelsnamen Cosmedia®Guar und Jaguar® vertriebenen Produkte,
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden,
- quaternierter Polyvinylalkohol,
   sowie die unter den Bezeichnungen
- Polyquaternium 2, Polyquaternium 17, Polyquaternium 18, Polyquaternium 27, Polyquaternium-32, Polyquaternium-37, Polyquaternium 67, Polyquaternium 74 und Polyquaternium 89 bekannten Polymere.

Gleichfalls als kationische Polymere eingesetzt werden können die unter den Bezeichnungen Polyquaternium-24 (Handelsprodukt z. B. Quatrisoft® LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat®ASCP 1011, Gafquat®HS 110, Luviquat®8155 und Luviquat® MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind

In einer bevorzugten Ausführungsform der Erfindung enthalten die Reinigungszusammensetzungen weiterhin mindestens ein zuvor genanntes kationisches quaternisiertes Cellulosepolymer, ein kationisches Guarderivat und/oder ein kationisches Polymer auf Acrylsäure(derivat)basis, die insbesondere ausgewählt sind aus den unter den INCI-Bezeichnungen bekannten Polymeren Polymer JR®- oder Celquat®, Jaguar®- oder N-Hance®-Polymeren, Polyquaternium-6, Polyquaternium-7, Polyquaternium-10, Polyquaternium-67, Polyquaternium 74 und/oder Polyquaternium 89.

Besonders bevorzugt sind erfindungsgemäß die quaternisierten Cellulosepolymere, die unter der INCI-Bezeichnung Polyquaternium-10 bekannt sind (Polymer JR®- oder Celquat®-Polymere). In einer zweiten besonders bevorzugten Ausführungsform der Erfindung enthalten die reinigenden Zusammensetzungen zur weiteren Verbesserung der Haarsensorik neben mindestens einem geradkettigen oder verzweigten Lauryl- und/oder Tridecylethersulfat mit einem Ethoxylierungsgrad von 2 bis 4, Cocamidopropylbetain, Cocoamphoacetate oder -diacetate und Cocamide MEA oder Cocamide DEA weiterhin mindestens ein zuvor beschriebenes kationisches quaternisiertes Cellulosederivat.

Die erfindungsgemäßen reinigenden Zusammensetzungen enthalten weiterhin 0,0025 bis 1 Gew.-%, vorzugsweise 0,005 bis 0,5 Gew.-% und insbesondere 0,01 bis 0,3 Gew.-% Jojobaöl.

Ein Zusatz von Jojobaöl erhöht die Pflegeeigenschaften der erfindungsgemäßen kosmetischen Reinigungszusammensetzungen.

Zur weiteren Stabilisierung des Jojobaöls können die erfindungsgemäßen Reinigungszubereitungen weiterhin ein nichtionisches Tensid mit einem HLB-Wert von 12 bis 18 enthalten, welches ethoxyliert ist.

Solche auch als Lösungsvermittler bezeichneten nichtionischen Tenside solubilisieren Jojobaöl in der Reinigungszubereitung, so dass je nach den Wünschen der Verbraucher transparente Reinigungszusammensetzungen hergestellt werden können. Unter transparenten Zusammensetzungen werden erfindungsgemäß bevorzugt solche Reinigungszusammensetzungen verstanden, die einen NTU-Wert (Nephelometrischer Trübungswert) von maximal 100, bevorzugt von maximal 50 und insbesondere von maximal 30 aufweisen.

Erfindungsgemäß geeignete ethoxylierte nichtionische Tenside eines HLB-Werts von 12 bis 18 sind beispielsweise
- Anlagerungsprodukte von 10 bis 50 Mol Ethylenoxid und/oder 0 bis 5 Mol Propylenoxid an lineare und verzweigte Fettalkohole mit 10 bis 30 C-Atomen,
- Anlagerungsprodukte von 5 bis 60 Mol Ethylenoxid an Rizinusöl und gehärtetes Rizinusöl und
- Sorbitanfettsäureester sowie Anlagerungsprodukte von 5 bis 100 Mol Ethylenoxid an Sorbitanfettsäureester wie beispielsweise die Polysorbate.

Erfindungsgemäß besonders geeignete Lösungsvermittler sind die Anlagerungsprodukte von 5 bis 60 Mol Ethylenoxid an Rizinusöl und gehärtetes Rizinusöl, insbesondere die unter der INCI-Bezeichnungen bekannten Lösungsvermittler PEG-40 Hydrogenated Castor Oil und PEG-60 Hydrogenated Castor Oil.

Die erfindungsgemaßen Reinigungszusammensetzungen weisen bevorzugt einen pH-Wert im Bereich von 3 bis 6, bevorzugt von 4 bis 5,5 und insbesondere von 4,7 bis 5,3 auf.

Neben den zuvor genannten Bestandteilen können die erfindungsgemäßen Zusammensetzungen eine Reihe Fakultativer Stoffe enthalten. Zu diesen fakultativen Inhaltsstoffen zählen weitere Tenside, die sich von den zuvor genannten Tensiden unterscheiden, Perlglanzmittel, Pflanzenextrakte, weitere Fett- oder Olkomponenten, die sich von den zuvor genannten Wachsen unterscheiden, sowie Vitamine und/oder Vitaminderivate.

Als weitere anionische Tenside eignen sich in erfindungsgemaßen Zubereitungen
- lineare und verzweigte Fettsauren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- sulfatierte Hydroxyalkylpolyethylen- und/oder Hydroxyalkylenpropylenglykolether,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (TI), 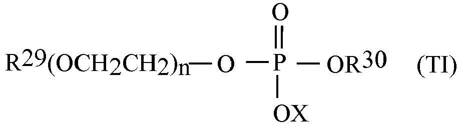 in der R²⁹ bevorzugt für einen aliphatischen Kohlenwasserstoffrest mit 8 bis 30 Kohlenstoffatomen, R³⁰ für Wasserstoff, einen Rest (CH₂CH₂O)ₙR²⁹ oder X, n für Zahlen von 1 bis 10 und X für Wasserstoff, ein Alkali- oder Erdalkalimetall oder NR³¹R³²R³³R³⁴, mit R³¹ bis R³⁴ unabhängig voneinander stehend für einen C₁ bis C₄ - Kohlenwasserstoffrest, steht,

Die fakultativen anionischen Tenside können in den erfindungsgemäßen Zubereitungen - bezogen auf deren Gesamtgewicht - bevorzugt in einer Menge von 0,05 bis 10 Gew.-%, mehr bevorzugt von 0,1 bis 7,5 Gew.-%, besonders bevorzugt von 0,2 bis 5 Gew.-% und insbesondere in einer Menge von 0,25 bis 3 Gew.-% eingesetzt werden.

Weiter fakultative nichtionische Tenside sind beispielsweise
- C₈-C₃₀-Fettsäuremono- und -diester von Anlagerungsprodukten von 1 bis 30 Mol Ethylenoxid an Glycerin,
- Aminoxide und
- Alkylpolyglucoside.

Alkylpolyglucoside entsprechen der Formel (I)

R¹O-[G]ₚ (I)

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Die Alkyl- und/oder Alkenyloligoglycoside können sich von Aldosen bzw. Ketosen mit 5 oder 6 Kohlenstoffatomen, vorzugsweise der Glucose ableiten. Die bevorzugten Alkyl- und/oder Alkenyloligoglycoside sind somit Alkyl- und/oder Alkenyloligoglucoside. Die Indexzahl p in der allgemeinen Formel (I) gibt den Oligomerisierungsgrad (DP), d.h. die Verteilung von Mono- und Oligoglycosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglycosid eine analytisch ermittelte rechnerische Größe, die meistens eine gebrochene Zahl darstellt. Vorzugsweise werden Alkyl- und/oder Alkenyloligoglycoside mit einem mittleren Oligomerisierungsgrad p von 1,1 bis 3,0 eingesetzt. Aus anwendungstechnischer Sicht sind solche Alkyl- und/oder Alkenyloligoglycoside bevorzugt, deren Oligomerisierungsgrad kleiner als 1,7 ist und insbesondere zwischen 1,2 und 1,7 liegt. Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen. Bevorzugt sind Alkyloligoglucoside der Kettenlänge C₈-C₁₀ (DP = 1-3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1-3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylakohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucyalkohol, Brassidylalkohol sowie deren technsiche Gemische, wie sie wie oben beschrieben erhalten werden können.

Für den Fall, dass ein weiteres nichtionisches Tensid in den Reinigungszusammensetzungen eingesetzt wird, sind Alkyloligoglucoside, insbesondere Alkyloligoglucoside auf der Basis von gehärtetem C_{12/14}-Kokosalkohol mit einem DP von 1-3, wie sie beispielsweise unter der INCI-Bezeichnung "Coco-Glucoside" im Handel erhältlich sind, bevorzugt.

Die fakultativen nichtionischen Tenside können in den erfindungsgemäßen Zubereitungen - bezogen auf deren Gesamtgewicht - bevorzugt in einer Menge von 0,05 bis 10 Gew.-%, mehr bevorzugt von 0,1 bis 7,5 Gew.-%, besonders bevorzugt von 0,2 bis 5 Gew.-% und insbesondere in einer Menge von 0,25 bis 3 Gew.-% eingesetzt werden.

Als fakultative Komponente können die erfindungsgemäßen Reinigungszubereitungen - bezogen auf ihr Gewicht - weiterhin 0,01 bis 3 Gew.-%, bevorzugt 0,025 bis 2 Gew.-% und insbesondere 0,05 bis 1 Gew.-% eines Perlglanzmittels enthalten.

Erfindungsgemäß geeignete Perlglanzmittel sind beispielsweise
- Glycoldistearinsäureester,
- C₈-C₃₀-Fettsäuremonoglycolester und
- mit Titandioxid überzogene Glimmerpigmente,
wie sie beispielsweise unter den Handelsbezeichnungen Rewopal®, Genapol® PMS, Cutina® EGMS, Timiron®, Colorona® und Euperlan® erhältlich sind.

Eine weitere Komponente, die in den erfindungsgemäßen Mitteln eingesetzt werden kann, ist ein Pflanzenextrakt.

Unter erfindungsgemäß geeigneten Pflanzenextrakten sind Extrakte zu verstehen, die aus allen Teilen einer Pflanze hergestellt werden können.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Erfindungsgemäß sind vor allem die Extrakte aus Grünem Tee, Eichenrinde, Brennessel, Hamamelis, Hopfen, Kamille, Klettenwurzel, Schachtelhalm, Weißdorn, Lindenblüten, Litschi, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Ginseng, Ingwerwurzel, Echinacea purpurea, Olea europea, Foeniculum vulgaris und Apim graveolens bevorzugt.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Zu den weiteren Fett- oder Ölkomponenten, die sich von den zuvor genannten Wachsen unterscheiden, zählen Silikone, Fettalkohole, Fettsäuren, Fettsäureester sowie natürliche Öle pflanzlichen und tierischen Ursprungs. Sie werden in den erfindungsgemäßen Reinigungsmitteln - bezogen auf ihr Gesamtgewicht - bevorzugt in einer Menge von 0,01 bis 10 Gew.-%, mehr bevorzugt von 0,05 bis 5 Gew.-% und insbesondere von 0,1 bis 3 Gew.-% eingesetzt.

Unter dem Begriff Silikonöle versteht der Fachmann mehrere Strukturen siliciumorganischer Verbindungen.

Geeignete Silikone enthalten mindestens ein Vertreter aus der Gruppe siliciumorganischer Verbindungen, die gebildet wird aus:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Silikonpolymeren mit nicht silikonhaltigem, organischen Grundgerüst, die aus einer organischen Hauptkette bestehen, welche aus organischen Monomeren gebildet wird, die kein Silikon enthalten, auf die in der Kette sowie gegebenenfalls an mindestens einem Kettenende mindestens ein Polysiloxanmakromer gepfropft wurde;
(v) gepfropften Silikonpolymeren mit Polysiloxan-Grundgerüst, auf das nicht silikonhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silikon enthält, wie beispielsweise das unter der INCI-Bezeichnung Bis-PEG/PPG-20/20 Dimethicone vertriebene Handelsprodukt Abil B 8832 der Firma Degussa;
(vi) oder deren Gemischen.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol® 871 und Emersol® 875, und Isopalmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Als Fettalkohole können eingesetzt werden gesättigte, ein- oder mehrfach ungesättigte, verzweigte oder unverzweigte Fettalkohole mit C₆-C₃₀-, bevorzugt C₁₀-C₂₂- und ganz besonders bevorzugt C₁₂-C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol®, z.B. Stenol® 1618 oder Lanette®, z.B. Lanette® O oder Lorol®, z.B. Lorol® C8, Lorol® C14, Lorol® C18, Lorol® C8-18, HD-Ocenol®, Crodacol®, z.B. Crodacol® CS, Novol®, Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können erfindungsgemäß auch Wollwachsalkohole, wie sie beispielsweise unter den Bezeichnungen Corona®, White Swan®, Coronet® oder Fluilan® käuflich zu erwerben sind, eingesetzt werden.

Zu den natürlichen und synthetischen kosmetischen Ölkörpern sind beispielsweise zu zählen:
- Esteröle. Unter Esterölen sind zu verstehen die Ester von C₆-C₃₀ - Fettsäuren mit C₂-C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit® IPM), Isononansäure-C₁₆-₁₈-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-dipelargonat, Butandiol-di-isostearat, Neopentylglykoldicaprylat,
- symmetrische, unsymmetrische oder cyclische Ester der Kohlensäure mit Fettalkoholen, Glycerincarbonat oder Dicaprylylcarbonat (Cetiol® CC),
- Trifettsäureester von gesättigten und/oder ungesättigten linearen und/oder verzweigten Fettsäuren mit Glycerin,
- Fettsäurepartialglyceride, das sind gegebenenfalls ethoxylierte Monoglyceride, Diglyceride und deren technische Gemische. Bei der Verwendung technischer Produkte können herstellungsbedingt noch geringe Mengen Triglyceride enthalten sein. Die Partialglyceride folgen vorzugsweise der Formel, 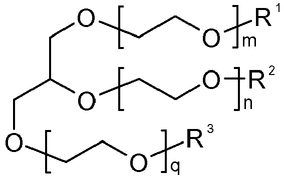 in der R1, R2 und R3 unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R1 für einen Acylrest und R2 und R3 für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Als natürliche Öle kommen beispielsweise Amaranthsamenöl, Aprikosenkernöl, Arganöl, Avocadoöl, Babassuöl, Baumwollsaatöl, Borretschsamenöl, Camelinaöl, Distelöl, Erdnußöl, Granatapfelkernöl, Grapefruitsamenöl, Hanföl, Hagebuttenkernöl, Haselnussöl, Holundersamenöl, Johannisbeersamenöl, Kakaobutter, Leinöl, Macadamianussöl, Maiskeimöl, Mandelöl, Marulaöl, Nachtkerzenöl, Olivenöl, Palmöl, Pfirsichkernöl, Rapsöl, Reisöl, Sanddornfruchtfleischöl, Sanddornkernöl, Sesamöl, Sheabutter, Sojaöl, Sonnenblumenöl, Traubenkernöl, Walnußöl oder Wildrosenöl in Frage.

Besonders bevorzugt sind Avocadoöl, Aprikosenkernöl, Hagebuttenkernöl, Kakaobutter, Mandelöl, Olivenöl, Pfirsichkernöl, Sheabutter, Sonnenblumenöl und Traubenkernöl.

Ebenfalls als vorteilhaft hat sich die Kombination der erfindungsgemäßen Zusammensetzung mit Vitaminen, Provitaminen und Vitaminvorstufen sowie deren Derivaten erwiesen.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht. Die Vitamin A-Komponente wird bevorzugt in Mengen von 0,05-1 Gew.-%, bezogen auf die gesamte Zubereitung, eingesetzt.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Bevorzugt ist das Nicotinsäureamid, das bevorzugt in Mengen von 0,05 bis 1 Gew.-%, bezogen auf das gesamte Mittel, eingesetzt wird.
- Vitamin B₅ (Pantothensäure und Panthenol). Im Rahmen dieser Gruppe wird bevorzugt das Panthenol eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate. Die genannten Verbindungen des Vitamin B₅-Typs werden bevorzugt in Mengen von 0,05 - 10 Gew.-%, bezogen auf das gesamte Mittel, eingesetzt. Mengen von 0,1 - 5 Gew.-% sind besonders bevorzugt.
- Vitamin B₆ (Pyridoxin sowie Pyridoxamin und Pyridoxal).
   Vitamin C (Ascorbinsäure). Die übliche Einsatzmenge von Vitamin C beträgt 0,1 bis 3 Gew.-%, bezogen auf das gesamte Mittel. Die Verwendung in Form des Palmitinsäureesters, der Glucoside oder Phosphate kann bevorzugt sein. Die Verwendung in Kombination mit Tocopherolen kann ebenfalls bevorzugt sein.
- Vitamin E (Tocopherole, insbesondere α-Tocopherol). Tocopherol und seine Derivate, worunter insbesondere die Ester wie das Acetat, das Nicotinat, das Phosphat und das Succinat fallen, werden bevorzugt in Mengen von 0,05-1 Gew.-%, bezogen auf das gesamte Mittel, eingesetzt.
- Vitamin F. Unter dem Begriff "Vitamin F" werden üblicherweise essentielle Fettsäuren, insbesondere Linolsäure, Linolensäure und Arachidonsäure, verstanden.
- Vitamin H. Als Vitamin H wird die Verbindung (3aS,4S, 6aR)-2-Oxohexahydrothienol[3,4-d]-imidazol-4-valeriansäure bezeichnet, für die sich aber zwischenzeitlich der Trivialname Biotin durchgesetzt hat. Biotin wird bevorzugt in Mengen von 0,0001 bis 1,0 Gew.-%, insbesondere in Mengen von 0,001 bis 0,01 Gew.-% eingesetzt.

In den erfindungsgemäßen Reinigungsmitteln werden bevorzugt Vitamine, Provitamine und Vitaminvorstufen aus den Gruppen A, B, E und H eingesetzt, wobei Panthenol, Pantolacton und Nicotinsäureamid besonders bevorzugt sind.

Bevorzugte Ausführungsformen der erfindungsgemäßen kosmetischen Reinigungszusammensetzung sind Shampoos, Duschbäder, Duschgele, Haarspülungen, Haarkuren, Rasierwässer und/oder Deodorantien. Insbesondere bevorzugt sind erfindungsgemäße Reinigungsmittel, die der Haar- und Kopfhautreinigung dienen.

Neben den erfindungsgemäß zwingenden Komponenten und den weiteren zuvor genannten bevorzugten Komponenten können die erfindungsgemäßen Reinigungszubereitungen weitere, dem Fachmann für solche kosmetischen Mittel bekannten Komponenten enthalten.

Unter diese fallen beispielsweise:
- Strukturanten wie Maleinsäure und Milchsäure,
- faserstrukturverbessernde Wirkstoffe, insbesondere Mono-, Di- und Oligosaccharide wie beispielsweise Glucose, Galactose, Fructose, Fruchtzucker und Lactose,
- Farbstoffe zum Anfärben des Mittels,
- Antischuppenwirkstoffe wie Piroctone Olamine, Zink Omadine, Zinkpyrithion und Climbazol,
- weitere Substanzen zur Einstellung des pH-Wertes, wie beispielsweise α- und β-Hydroxycarbonsäuren,
- Wirkstoffe wie Allantoin und Bisabolol,
- Komplexbildner wie EDTA, NTA, β-Alanindiessigsäure und Phosphonsäuren,
- Treibmittel wie Propan-Butan-Gemische, N₂O, Dimethylether, CO₂ und Luft,
- Konsistenzgeber wie Zuckerester, Polyolester oder Polyolalkylether,
- Konservierungsmittel, wie beispielsweise Natriumbenzoat oder Salicylsäure,
- Viskositätsregler wie Salze (NaCl).

Ein zweiter Gegenstand der Erfindung ist ein Verfahren zur Haarbehandlung, bei dem eine erfindungsgemäße kosmetische Reinigungszusammensetzung auf die nassen Haare aufgebracht, und nach einer Einwirkungszeit von 30 Sekunden bis 5 Minuten mit Wasser ausgespült wird.

### Beispiele:

Es wurden die folgenden Reinigungszusammensetzungen hergestellt.

Die Zusammensetzung E1 ist erfindungsgemäß; die Zusammensetzungen V1, V2 und V3 sind Vergleichszusammensetzungen.

| | **E1** | **V1** | **V2** | **V3** |
|---|---|---|---|---|
| Natriumlaurylethersulfat (2EO) | 8,750 | 10,780 | 10,780 | 9,80 |
| NaOH | 0,059 | 0,0735 | 0,0735 | 0,067 |
| Salicylsäure | 0,200 | 0,200 | 0,200 | 0,200 |
| Citronensäure | 0,200 | 0,500 | 0,1500 | 0225 |
| Sieben Kräuter Extrakt®⁷ | 0,100 | 0,100 | 0,100 | 0,100 |
| Natriumbenzoat | 0,500 | 0,500 | 0,500 | 0,500 |
| Euperlan PK 3000 AM®¹ | 2,350 | 2,600 | 2,600 | 2,350 |
| D-Panthenol | 0,150 | 0,150 | 0,150 | 0,150 |
| Nicotinsäureamid | 0,150 | 0,150 | 0,150 | 0,150 |
| Disodium Cocoamphodiacetate | 2 | 7 | | |
| Jojobaöl goldgelb DAB | 0,010 | | | |
| PEG-40 Hydrogenated Castor Oil 455 | 0,100 | | | |
| Cetiol HE®² | 0,300 | 0,300 | 0,300 | 0,300 |
| Cutina HR®³ | 0,100 | 0,100 | 0,100 | 0,100 |
| Comperlan 100®⁴ | 0,450 | | | 0,450 |
| Polymer JR®⁵ 400 | 0,080 | 0,100 | 0,100 | 0,080 |
| Cocamidopropylbetain | 1,600 | | 2,400 | 2,040 |
| Natriumchlorid | 1,100 | 1,500 | 1,500 | 1,500 |
| Farbstoff, Parfum | q.s. | q.s. | q.s. | q.s. |
| Wasser | ad 100 | ad 100 | ad 100 | ad 100 |

Es wurden die folgenden Handelsprodukte verwendet:
1 INCI-Bezeichnung: Wasser, Glycol Distearate, Glycerin, Laureth-4, Cocamidopropylbetain; Cognis,
2 INCI-Bezeichnung: PEG-7 Glyceryl Cocoate; Cognis,
3 INCI-Bezeichnung: Hydrogenated Castor Oil; Cognis,
4 INCI-Bezeichnung: Cocamide MEA; Cognis,
5 INCI-Bezeichnung: Polyquaternium-10, Dow (Amerchol),
6 INCI-Bezeichnung: Hydroxypropyl Guar Hydroxypropyltrimonium Chloride; Rhodia
7 INCI-Bezeichnung: Propylene Glycol, Wasser, Chamomilla Recutita (Matricaria) Flower Extract, Lactic Acid, Rosmarinus Officialis (Rosemary) Leaf Extract, Equisetum Arvenese Extract, Achillea Millefolinium Extract, Thymus Vulgaris (Thyme) Extract, Hayflower Extract, Thymus Serpyllum Extract; Croda

Die zuvor genannten fünf Zusammensetzungen wurden neutral verpackt, codiert und sechs Wochen lang von jeweils 150 Verwendern getestet.

Die Ausgewogenheit der Shampoos im Hinblick auf die Haarsensorik nach der Reinigung, das Schaumvermögen und die Textur des Shampoos wurde beurteilt.

Die Verwender bewerteten die Shampoos mit den Symbolen -, 0 und +, die für

| | |
|---|---|
| "-" | nicht zufrieden, |
| "0" | akzeptabel und |
| "+" | sehr zufrieden |

stehen.

Die Auswertung ergab die folgenden Ergebnisse:

| | **E1** | **V1** | **V2** | **V3** |
|---|---|---|---|---|
| **-** | 3 | 8 | 9 | 6 |
| **0** | 28 | 37 | 41 | 33 |
| **+** | 119 | 105 | 100 | 111 |

## Patentansprüche

1. Reinigende kosmetische Zusammensetzung, enthaltend in einem kosmetischen Träger - bezogen auf ihr Gewicht -
a) 3 bis 20 Gew.-% mindestens eines anionischen Tensids,
b) 0,05 bis 5 Gew-% mindestens eines Tensids der Formel (iii), 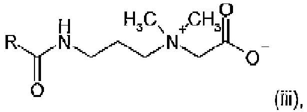 in der R für einen geradkettigen oder verzweigten, gesättigten oder ein- bzw. mehrfach ungesättigten Alkyl- oder Alkenlyrest mit 8 bis 24 Kohlenstoffatomen steht,
c) 0,05 bis 5 Gew.-% mindestens eines Tensids der Formel (v), 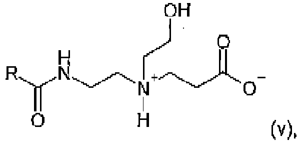 in der R für einen geradkettigen oder verzweigten, gesättigten oder ein- bzw. mehrfach ungesättigten Alkyl- oder Alkenlyrest mit 8 bis 24 Kohlenstoffatomen steht.
d) 0,01 bis 5 Gew.-% mindestens eines C₂-C₄-Mono- und/oder Dialkanolamids mindestens einer C₆-C₂₄-Carbonsäure und
e) 0,0025 bis 1 Gew.-% mindestens eines pflanzlichen, mineralischen, synthetischen oder tierischen Wachses,
wobei das Wachs e) Jojobaöl ist.

2. Zusammensetzung nach Anspruch 1, **dadurch gekennzeichnet, dass** sie mindestens ein anionisches Tensid enthält, das ein geradkettiges oder verzweigtes Alkylsulfonat mit einem Alkylrest von 8 bis 18, insbesondere von 10 bis 16 C-Atomen, und/oder ein geradkettiges oder verzweigtes Alkylethersulfat mit einem Alkylrest von 8 bis 18, insbesondere von 10 bis 16 C-Atomen und mit 1 bis 6, insbesondere mit 2 bis 4, Ethylenoxideinheiten ist,

3. Zusammensetzung nach einem der Ansprüche 1 oder 2, **dadurch gekennzeichnet, dass** sie - bezogen auf ihr Gewicht - vorzugsweise 0,1 bis 4 Gew.-% und insbesondere 0,25 bis 2 Gew-% Tensid(e) der Formel (iii) und vorzugsweise 0,1 bis 4 Gew.-% und insbesondere 0,25 bis 2 Gew.-% Tensid(e) der Formel (v) enthält.

4. Zusammensetzung nach einem der Ansprüche 1 bis 3, **dadurch gekennzeichnet, dass** sie mindestens ein Mono- und/oder Diethanolamid und/oder mindestens ein Mono- und/oder Diisopropanolamid einer C₈-C₂₄-Fettsäure enthält, wobei die Fettsäure(n) Kokosfettsäuren, Laurinsäure, Myristinsäure, Palmitinsäure und/oder Stearinsäure ist (sind).

5. Zusammensetzung nach einem der Ansprüche 1 bis 4, **dadurch gekennzeichnet, dass** sie - bezogen auf ihr Gewicht-zusätzlich 0,01 bis 2 Gew.-%, vorzugsweise 0,02 bis 1 Gew.-% und insbesondere 0,025 bis 0,8 Gew.-% mindestens eines kationischen Polymeren enthält.

6. Zusammensetzung nach Anspruch 5, **dadurch gekennzeichnet, dass** mindestens ein kationisches Polymer ein quaternisiertes Cellulosederivat ist.

7. Zusammensetzung nach einem der Ansprüche 1 bis 6, **dadurch gekennzeichnet, dass** siebezogen auf ihr Gewicht - vorzugsweise 0,005 bis 0,5 Gew.-% und insbesondere 0,01 bis 0,3 Gew.-% eines pflanzlichen, mineralischen, synthetischen oder tierischen Wachses enthält, wobei das Wachs e) Jojobaöl ist.

8. Zusammensetzung nach einem der Ansprüche 1 bis 7, **dadurch gekennzeichnet, dass** sie zusätzlich ein ethoxyliertes nichtionisches Tensid mit einem HLB-Wert von 12 bis 18 enthält.

9. Verfahren zur Haarbehandlung, **dadurch gekennzeichnet, dass** eine kosmetische Reinigungszusammensetzung nach einem der Ansprüche 1 bis 8 auf die nassen Haare aufgebracht, und nach einer Einwirkungszeit von 30 Sekunden bis 5 Minuten mit Wasser ausgespült wird.

## Claims

1. A cleansing cosmetic composition containing, in a cosmetic carrier and based on its weight,
a) from 3 to 20 wt.% of at least one anionic surfactant,
b) from 0.05 to 5 wt.% of at least one surfactant of formula (iii), 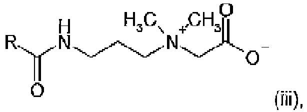 in which R denotes a straight-chain or branched, saturated or mono- or polyunsaturated alkyl or alkenyl functional group having from 8 to 24 carbon atoms,
c) from 0.05 to 5 wt.% of at least one surfactant of formula (v), 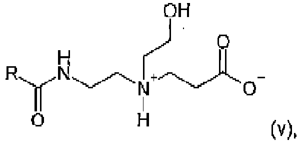 in which R denotes a straight-chain or branched, saturated or mono- or polyunsaturated alkyl or alkenyl functional group having from 8 to 24 carbon atoms,
d) from 0.01 to 5 wt.% of at least one C₂-C₄ mono- and/or dialkanolamide of at least one C₈-C₂₄ carboxylic acid and
e) from 0.0025 to 1 wt.% of at least one vegetable, mineral, synthetic or animal wax, wherein the wax e) is jojoba oil.

2. The composition according to claim 1, **characterized in that** it contains at least one anionic surfactant, which is a straight-chain or branched alkyl sulfonate comprising an alkyl functional group having from 8 to 18, in particular from 10 to 16 carbon atoms, and/or a straight-chain or branched alkyl ether sulfate comprising an alkyl functional group having from 8 to 18, in particular from 10 to 16 carbon atoms and comprising from 1 to 6, in particular from 2 to 4 ethylene oxide units.

3. The composition according to one of claims 1 or 2, **characterized in that** it contains, based on its weight, preferably from 0.1 to 4 wt.% and in particular from 0.25 to 2 wt.% surfactant(s) of formula (iii) and preferably from 0.1 to 4 wt.% and in particular from 0.25 to 2 wt.% surfactant(s) of formula (v).

4. The composition according to one of claims 1 to 3, **characterized in that** it contains at least one mono- and/or diethanolamide and/or at least one mono- and/or diisopropanolamide of a C₈-C₂₄ fatty acid, the fatty acid(s) being coconut fatty acids, lauric acid, myristic acid, palmitic acid and/or stearic acid.

5. The composition according to one of claims 1 to 4, **characterized in that** it additionally contains, based on its weight, from 0.01 to 2 wt.%, preferably from 0.02 to 1 wt.% and in particular from 0.025 to 0.8 wt.% of at least one cationic polymer.

6. The composition according to claim 5, **characterized in that** at least one cationic polymer is a quaternized cellulose derivative.

7. The composition according to one of claims 1 to 6, **characterized in that** it contains, based on its weight, preferably from 0.005 to 0.5 wt.% and in particular from 0.01 to 0.3 wt.% of a plant, mineral, synthetic or animal wax, the wax e) being jojoba oil.

8. The composition according to one of claims 1 to 7, **characterized in that** it additionally contains an ethoxylated non-ionic surfactant having a HLB value of from 12 to 18.

9. A method for hair treatment, **characterized in that** a cosmetic cleansing composition according to one of claims 1 to 8 is applied to wet hair, and is rinsed out using water after a contact time of from 30 seconds to 5 minutes.

## Revendications

1. Composition cosmétique nettoyante, contenant, par rapport à son poids, dans un excipient cosmétique,
a) 3 à 20 % en poids d'au moins un tensioactif anionique,
b) 0,05 à 5 % en poids d'au moins un tensioactif de formule (iii), 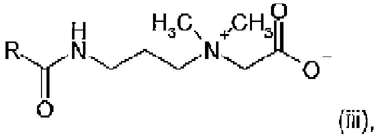 dans laquelle R représente un radical alkyle ou alcényle à chaîne droite ou ramifié, saturé ou une ou plusieurs fois insaturé, comportant 8 à 24 atomes de carbone,
c) 0,05 à 5 % en poids d'au moins un tensioactif de formule (v), 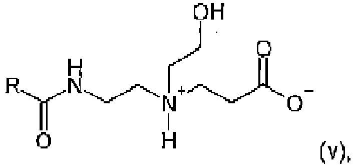 dans laquelle R représente un radical alkyle ou alcényle à chaîne droite ou ramifié, saturé ou une ou plusieurs fois insaturé, comportant 8 à 24 atomes de carbone,
d) 0,01 à 5 % en poids d'au moins un mono et/ou dialcanolamide en C₂-C₄ d'au moins un acide carboxylique en C₈-C₂₄ et
e) 0,0025 à 1 % en poids d'au moins une cire d'origine végétale, minérale, synthétique ou animale,
la cire e) étant de l'huile de Jojoba.

2. Composition selon la revendication 1, **caractérisée en ce qu'**elle contient au moins un tensioactif anionique, qui est un sulfonate d'alkyle à chaîne droite ou ramifié comportant un radical alkyle de 8 à 18, en particulier de 10 à 16 atomes C, et/ou un sulfate d'éther d'alkyle à chaîne droite ou ramifié comportant un radical alkyle de 8 à 18, en particulier de 10 à 16 atomes C, et comportant 1 à 6, en particulier 2 à 4 unités d'oxyde d'éthylène.

3. Composition selon une des revendications 1 ou 2, **caractérisée en ce qu'**elle contient, par rapport à son poids, de préférence 0,1 à 4 % en poids et en particulier 0,25 à 2 % en poids de tensioactif(s) de formule (III) et de préférence 0,1 à 4 % en poids et en particulier 0,25 à 2 % en poids de tensioactif(s) de formule (V).

4. Composition selon une des revendications 1 à 3, **caractérisée en ce qu'**elle contient au moins un mono et/ou diéthanolamide et/ou au moins un mono et/ou diisopropanolamide d'un acide gras en C₈-C₂₄, le ou les acides gras étant des acides gras de coco, l'acide dodécanoïque, l'acide myristique, l'acide palmitique et/ou l'acide stéarique.

5. Composition selon une des revendications 1 à 4, **caractérisée en ce qu'**elle contient, par rapport à son poids, en plus 0,01 à 2 % en poids, de préférence 0,02 à 1 % en poids et en particulier 0,025 à 0,8 % en poids d'au moins un polymère cationique.

6. Composition selon la revendication 5, **caractérisée en ce qu'**au moins un polymère cationique est un dérivé de cellulose quaternaire.

7. Composition selon une des revendications 1 à 6, **caractérisé en ce qu'**elle contient, par rapport à son poids, de préférence 0,005 à 0,5 % en poids et en particulier 0,01 à 0,3 % en poids d'une cire d'origine végétale, minérale, synthétique ou animale, la cire e) étant de l'huile de Jojoba.

8. Composition selon une des revendications 1 à 7, **caractérisée en ce qu'**elle contient, en plus, un tensioactif non ionique éthoxylé avec une valeur EHL de 12 à 18.

9. Procédé de traitement des cheveux, **caractérisé en ce qu'**une composition cosmétique nettoyante selon une des revendications 1 à 8 est appliquée sur les cheveux mouillés et rincé avec de l'eau après un temps d'action de 30 secondes à 5 minutes.
